# EUROPEAN PATENT APPLICATION

(11) **EP 1 939 961 A1**
(43) Date of publication of application: **02.07.2008**
(21) Application number: 06807958.1
(22) Date of filing: 27.09.2006
(51) Int. Cl.: H01M 4/88, H01M 8/16, C12N 11/14

(54) **BIOLOGICAL ELECTRODE WITH THE HYDROGENASE ENZYME, METHOD OF OBTAINING SAME AND APPLICATIONS THEREOF**

(30) Priority: 30.09.2005 ES 200502386
(71) Applicant: Consejo Superior de Investigaciones Cientificas (CSIC), 28006 Madrid (ES)
(72) Inventor: FERNÁNDEZ LÓPEZ, Victor Manuel, E-28049 Madrid (ES); LÓPEZ DE LACEY, Antonio, E-28049 Madrid (ES); RÜDIGER, Olaf, E-28049 Madrid (ES)
(74) Representative: Pons Arino, Angel
(86) International application number: PCT/ES2006/070139
(87) International publication number: WO 2007/039661

(57) **Abstract**

The invention relates to biological electrodes which are modified with hydrogenase enzymes (anodes) and which can be used to obtain electrical power from hydrogen in a standard fuel cell configuration. In addition, with said hydrogenase-modified electrodes (cathodes) it is possible to produce hydrogen from water in a standard electrochemical cell configuration. The invention also relates to method of producing same and to the applications thereof.

## Description

### TECHNICAL SECTOR OF THE INVENTION

The present invention relates to biological electrodes for hydrogen fuel cells as an alternative to other hydrogen oxidation catalysts such as platinum and other metals, and also for hydrogen generation in electrolytic cells. Therefore, it is related to biotechnology, genetic engineering and, more specifically, to the use of enzymes as biocatalysts and, more specifically, as redox biocatalysts, and, more specifically, to the biotechnology of hydrogen production and utilisation.

### STATE OF THE ART

At present, there is a lively debate regarding the type of fuel that will be used in the future to sustain human activity, which has successively gone from using primarily wood to using coal and, later, oil and natural gas, within a relatively short time span. The current trend is to diversify the sources, with a progressive use of methane and attention placed on renewable energies, amongst which hydrogen obtained from water is considered by some to be an "inexhaustible" energy source, although at present petroleum continues to be the raw material wherefrom the hydrogen we consume is obtained. Jointly with the nature of the fuel to be used, the most convenient way to use it is debated; in this respect, it is worth mentioning that, in any scenario that we may consider, and regardless of the fuel used, Fuel Cells are being chosen as one of the safest, cleanest, most efficient ways to transform the energy contained in those fuels into electricity (M.S. Dreseselhaus and I.L. Thomas, Alternative energy technologies. Nature, 414, 332- 337 (2001)). Within this context, it is worth mentioning that in the past few years we have witnessed intense research on Fuel Biocells, so called because biological elements, redox enzymes or microbial cells intervene in the cells' electrodes, in the anodes and cathodes (G. Tayhas, R. Palmore, and G.M. Whitesides, Microbial and Enzymatic Biofuel Cells in Enzymatic conversion of biomass for fuel production, M.E. Himmel, J.O. Baker and R.P. Overend, eds., American Chemical Society Symposium series, no. 566, ACS, Washington, DC., 271-290, 1994). In a recent review, A. Heller discusses the state of research in the field of enzymatic fuel biocells, the majority of which use glucose and oxygen as fuel. They are generally based on electrodes modified with the glucose oxidase and laccase enzymes, in such a way that, respectively, the glucose is oxidised in the anode and the consequent reduction of oxygen to water takes place in the cathode. One must bear in mind that redox mediators (in solution or in the form of redox polymers) tend to be used in these fuel biocells to transport electrons between the active centres of the respective enzymes and the electrodes (A. Heller, Miniature biofuel cells, Phys. Chem. Chem. Phys., 6, 209-216, 2004).

The first fuel biocell to use hydrogen and oxygen, described by S. Tsujimura (S. Tsujimura, M. Fujita, H. Tatsumi, K. Kano, T. Ikeda, Bioelectrocatalysis-based dihydrogen/dioxygen fuel cell operating at physiological pH, Phys. Chem. Chem. Phys., 3,1331-1335, 2001), uses *Desulfovibrio vulgaris* bacterial cells as the hydrogen consumption catalyst in the anode; furthermore, it adds the compound methyl viologen as redox mediator between the bacteria and the electrode.

More recently, a patent has been published (United States Patent Application 20040214053, A1, October 28, 2004, Foreign Application data: Aug 24,2001, GB, no. 0120698.6) about a biological fuel biocell that uses hydrogen as the fuel, wherein the anode is coated with purified type Ni-Fe hydrogenase produced from extracts of the *Allochromatium vinosum* bacteria. In relation to our patent, it is very significant that, in this invention, the enzyme is deposited on the carbon electrode by adsorption in the presence of cationic polymers; this resulted in functional electrodes, but they were not very stable. Previously, scientific articles had been published wherein hydrogenase enzymes from different microorganisms were deposited on electrodes by means of different types of non-covalent bonds:
(a) By adsorption on glassy carbon, with or without the addition of B polymyxin sulfate (Butt, J. N.; Filipiak, M.; Hagen, W. R. Direct Electrobiochemistry of Megasphaera elsdenii iron hydrogenase: Definition of the enzyme's catalytic operating potential and quantitation of the catalytic behaviour over a continuous potential range. Eur. J. Biochem. 245, 116-122, 1997). In this document, electrical currents are obtained in a hydrogen atmosphere, but the currents are not persistent; this they attribute either to protein loss or a non-favourable orientation of the enzyme's redox centres with respect to the surface of the electrode;
(b) by adsorption on "edge" pyrolytic graphite with the addition of B polymyxin sulfate (Pershad, H.R., Duff, J.L.D., Heering, H. A., Duin, E. C., Albracht, S.P.J., Armstrong, F.A. Catalytic electron transport in Allochromatium vinosum [Ni-Fe]-hydrogenase: Application of voltametry in detecting redox-active centres and establishing that hydrogen oxidation is very fast even at potentials close to the reversible H+/H2 value. Biochemistry, 38, 8992-8999, 1999). These authors show that the Ni-Fe hydrogenase under study, which is very similar to the one used in this invention, is extremely active, even though they do not know whether it has more than one enzyme layer and they do not know the orientation, contrary to us, since, using our method, a single monolayer is produced with the molecules in the favourable orientation for the exchange of current with the electrode to take place.
(c) Enzyme electrokinetics: Hydrogen evolution and oxidation by *Allochromatium vinosum* [Ni-Fe]-hydrogenase. Leger, C.; Jones, A.K.; Roseboom, W.; Albracht, S.P.J.; Armstrong, F.A. Biochemistry. 41, 15736-15746, 2002. In this document, the same electrode is used as in the preceding work: "edge" pyrolytic graphite by adsorption.
(d) Direct and electrically wired bioelectrocatalysis by hydrogen from *Thiocapsa roseopersicina.* Morozov, S.V.; Karyakina, E.E.; Zorin, N.A.; Varfolomeyev, S.D.; Cosnier, S.; Karyakin, A.A. Bioelectrochemistry 55, 169-171, 2002. Here, glassy carbon and carbon filaments are used, by physical adsorption.
(e) Inhibition and aerobic kinetics of *Desulfovibrio fructosovorans* NiFe hydrogenase studied by protein film voltammetry. Leger, C.; Dementin, S.; Bertrand, P.; Rousset, M.; Guigliarelli, B. J. Am. Chem. Soc. 126, 12162-12172, 2004. Here, "edge" pyrolytic graphite by physical adsorption is used.
(f) Hydrogenases from the hyperthermophilic bacterium Aquilex aeolicus: electrocatalysis of the hydrogen production/consumption reactions at carbon electrodes. Lojou, E.; Giudici-Orticoni, M.T.; Bianco, P. J. Electroanal. Chem., 577, 79-86, 2005. It is performed on graphite with hyperthermophillic enzymes; they do not study the stability, but there is no covalent bond.
(g) Design and characterization of redox enzyme electrodes: new perspectives on established techniques with application to an extremophile hydrogenase. Johnston, W.; Cooney, M.J.; Liaw, B.Y.; Sapra, R.; Adams, M.W.W. Enzyme Microb. Technol., 36, 540-549, 2005. These authors deposit a hyperthermophillic enzyme on porous pyrolytic carbon paper and column-packed graphite, also by direct adsorption. In all cases where they studied the stability of the electrodes, the electrocatalytic responses were not persistent and the authors have attributed this decrease in the electrodes' catalytic activity to the loss of hydrogenase enzyme from the surface of the electrode, where it was maintained through weak interactions, primarily electrostatic, or to movement of the hydrogenase adsorbed by other species in the solution.

In summary, other works do not include enzyme stability studies, and none of the published works perform immobilisation of the hydrogenase enzyme by covalent binding, as disclosed for the first time in this patent.

As an electrochemical support for the immobilisation of hydrogenase in particular, and proteins in general, different forms of carbon may be used, including glassy carbon, pyrolytic carbon or graphite, activated with reactive surface groups. A very convenient method to activate the carbon surface is the electrochemical reduction of aryl-diazonium salts developed by Pinson, Saveant et al. (J. Pinson and F. Podvorica, "Attachment of organic layers to conductive or semiconductive surfaces by reduction of diazonium salts" Chemical Society Reviews ,43, 429-439, 2005), which is protected by patents W09213983, US6217740, US6435240 and US2002144912, as is the use thereof to immobilise enzymes by covalent binding on carbon surfaces. Nevertheless, none of these patents or publications considers the oriented immobilisation of redox enzymes for direct electron transfer.

### DESCRIPTION

### BRIEF DESCRIPTION

An object of this invention is a biological electrode, hereinafter biological electrode of this invention, based on the use of a hydrogenase-type enzyme, of the Ni-Fe or "Fe-only" types, as a hydrogen oxidation catalyst, covalently bound, with a preferred orientation, to an electrode made of an electricity-conducting material through, respectively, carboxylic residues or amino residues exposed on the surface thereof or introduced in the surface of said electrodes by suitable chemical methods.

Another particular object of the invention is the biological electrode of this invention used as an electrochemical catalyst for the production of hydrogen.

Another particular object of the invention is the biological electrode of the invention wherein the hydrogenase enzyme contains affinity motifs located on specific sites of the surface thereof that allow for oriented immobilisation on the conducting electrode. For example, certain metal complexes have an affinity for histidine groups that may be introduced in hydrogenase by means of genetic engineering techniques.

Thus, a particular object of this invention is the biological electrode of the invention wherein the conducting electrode material used is a metallic material that belongs, for illustrative purposes and without it limiting the scope of the invention, to the following group: gold, copper, silver or platinum.

Another particular object of this invention is the biological electrode of the invention wherein the electrode material used is a carbonaceous material that belongs, for illustrative purposes and without this limiting the scope of the invention, to the following group: glassy carbon, "basal" pyrolytic carbon, "edge" pyrolytic carbon, carbon thread and carbon fabric.

A particular embodiment of this invention is the biological electrode of the invention wherein the hydrogenase-type enzyme is isolated from the *Desulfovibrio gigas* E.C.1.18.89.1 bacteria (the aa sequence of the enzyme used has the PDB reference code 1 H2A in the Protein Data Bank) and the electrode is gold (see example 1).

Another particular embodiment of this invention is the biological electrode of the invention wherein the hydrogenase-type enzyme is isolated from the *Desulfovibrio gigas* E.C.1.18.89.1 bacteria (the aa sequence of the enzyme used has the PDB reference code 1H2A in the Protein Data Bank) and the electrode is made of "edge" pyrolytic carbon (see example 2).

Another particular embodiment of the invention considers that the biological electrode of the invention is composed of a carbonaceous material electrode, modified with carboxyl groups and additionally modified with N,N-bis(carboxymethyl)-L-lysine, to form nitrilotriacetic-type complexes with Ni, Cu or Co ions (these metal complexes have an affinity for histidine groups), and a modified or mutated hydrogenase enzyme which comprises a histidine motif on suitable sites of the surface of the enzyme, thus allowing for correct immobilisation of the hydrogenase molecules with respect to the electrode.

Another object of this invention is a method of producing the biological electrode of the invention with the conducting electrode made of carbon material, hereinafter method of producing a carbon electrode of the invention, based on the following steps:
a) modification of the carbon electrode by electroreduction of an aryl derivative of diazonium salts in an aprotic medium or an acid aqueous medium, which generates primary amino groups,
b) covalent binding of the hydrogenase molecules in phosphate buffer between 0.01 and 1.0 M, pH between 5.0 and 9.0, through its carboxyl groups activated with N-hydroxysuccinimide in the presence of soluble ethylcarbodiimide during a suitable period of time, and
c) subsequent washing of the electrode with phosphate buffer.
   Another particular object of this invention is the method of producing a carbon electrode of the invention wherein the aryl derivative of the diazonium salts in point a) belongs, for illustrative purposes and without this limiting the scope of the invention, to the following group: nitrobenzyldiazonium salt and 2,5-dimethoxy-4-([4-nitrophenyl]azo)benzenediazonium.

A particular embodiment of the invention is the method of producing the invention wherein the covalent binding step in b) is performed in 0.01 M phosphate buffer, pH 6.0, in the presence of 1 mM N-hydroxysuccinimide and 2 mM ethylcarbodiimide, for an incubation period of 90 minutes.

Another particular embodiment of the invention is a method of producing the carbon electrode wherein the electrode is an "edge" pyrolytic carbon electrode previously modified with the reagent tetrafluoroborate 4-nitrobenzenediazonium (according to the method described by P. Allonge et al. in the Journal of the American Chemical Society, 119, 201-207, 1997), which is put in contact with a 27 µM solution of purified hydrogenase from *Desulfovibrio gigas* in 0.01 M phosphate buffer, pH 6.0, in the presence of 1 mM N-hydroxysuccinimide and 2 mM ethylcarbodiimide, for an incubation period of 90 minutes in this solution, with subsequent washing of the electrode with phosphate buffer. This biological electrode is activated in a hydrogen atmosphere at ambient temperature for 5 hours.

Another object of this invention is a second method of producing the carbon electrode of the invention, which is based on the modification of carbon material surfaces by plasma irradiation to generate carboxyls which, conveniently modified with diamines, for example in the presence of carbodiiimide, and eventually N-hydroxysuccinimide, lead to coatings with primary amines.

Another object of this invention is a method of producing the biological electrode of the invention with the metallic conducting electrode, hereinafter method of producing a metallic electrode of the invention, by modification of the metal, for example gold, which consists of a coating made of a monolayer of bifunctional alkylthiol molecules that have, on one end, thiol groups with chemisorption tendencies and, on the other end, functional groups that react with exposed functional groups on the hydrogenase enzyme to produce the oriented immobilisation thereof.

Another object of this invention is the use of the biological electrode of the invention, hereinafter use of the invention, in the manufacturing of a fuel cell anode or an electrochemical cell cathode. Similarly, a fuel cell or an electrolytic cell that comprise the biological electrode of the invention also form a part of this invention.

### DETAILED DESCRIPTION

This invention addresses the problem of providing new biological electrodes for fuel cells which are stable under operating conditions.

This invention discloses, for the first time, the production of a stable, effective biological electrode by means of the covalent binding of surface carboxylic residues on the hydrogenase enzyme exposed on the surface of the enzyme, with primary amines introduced, by suitable chemical methods, in the surface of a metallic or carbonous material electrode that serves as anchoring for the enzymes. A noteworthy result of the research conducted, which is an object of this invention, has been the development of an effective method for the oriented immobilisation of hydrogenase enzyme molecules from *Desulfovibrio gigas* on the surface of an electrode made, for example, of pyrolytic carbon modified with reactive chemical compounds such that they covalently bind the enzyme molecules with such an orientation that electrical currents are generated when there is hydrogen in the atmosphere. In the absence of the enzyme, or with the electrode unmodified, said currents are not observed. Moreover, these biological electrodes also catalyse the production of hydrogen when a suitable cathode potential is applied (Example 2).

Furthermore, the method of immobilising (or anchoring) the hydrogenase molecules disclosed in this invention makes it possible to produce a biological electrode wherein the enzyme molecules are optimally oriented on the surface of the electrode, such that they may catalyse the oxidation of the hydrogen molecule, or the reduction of protons, without the need to incorporate redox mediators, either soluble or immobilised, monomeric or polymeric, into the system. Using this method, the hydrogenase molecules are bound to the surface of the electrode in an oriented manner, as a function of the pH and the ionic strength of the coupling reaction medium (Example 2).

The enzyme used exhibits binding motifs with specific affinity for other motifs generated on the surface of the electrode, such that, following interaction between both motifs, whether the bond is covalent, electrostatic or van der Waals, it adopts a preferred orientation with respect to the surface of the electrode that facilitates the transfer of electrons between both entities, the electrode and the enzyme; this preferred orientation is consolidated by chemical crossover through the respective enzyme-electrode chemical functional groups such that the enzyme is firmly anchored on the surface of the electrode in a preferred orientation of its redox centres that facilitates direct transfer of electrons between the supporting electrode and the enzyme.

These biocatalysts allow for the generation of electrical currents from hydrogen and the electrolytic generation of hydrogen from water using hydrogenases as the electrochemical catalyst, which is also an alternative to the use of non-biological catalysts. The use of these biological catalysts, capable of activating the hydrogen molecule, in hydrogen fuel cells could compete with other catalysts that are commonly used to oxidise hydrogen in the fuel cell anode, or to generate hydrogen in the electrochemical cell cathode, due to the high price of these noble metals and/or their not being too abundant, whereas hydrogenases are inexhaustible because they are produced in living organisms that may be perpetuated by biological reproduction. These aspects are important because the price of the metals currently used for those purposes is expected to increase considerably as the use of hydrogen as a clean fuel expands. On the contrary, the production of hydrogenases, both those commonly known as "Fe-Ni" and those known as "Fe-only", is a process that is susceptible to biotechnological improvements, with the consequent decrease in the price of the enzymes produced by microbial or algae cultivation.

Therefore, an object of this invention is a biological electrode, hereinafter biological electrode of this invention, based on the use of a hydrogenase-type enzyme, either Ni-Fe or "Fe-only" type, as a catalyst for the oxidation of hydrogen, covalently bound to an electrode made of an electricity-conducting material through, respectively, carboxylic residues or amino residues exposed on the surface thereof or introduced in the surface of said electrodes by suitable chemical methods.

The term "hydrogenase-type enzyme", as used in this invention, refers to an Ni-Fe-type or Fe-only-type hydrogenase isolated from hydrogen-activating microorganisms - specially bacteria, fungi and algae -, so called because of the nature of its active centre, which is, respectively, a group formed by a Fe ion with another Ni ion or only Fe ions, in addition to other non-metallic elements such as sulfur, carbon, nitrogen and oxygen ("Hydrogen as a fuel: learning from nature". R. Cammack, M. Fery and R. Robson, editors. Taylor and Francis, London and New York, 2001, "Hydrogenases", Coordination Chemistry Reviews, 249, numbers 15 and 16, 2005). Moreover, this term includes those hydrogenase-type enzymes or peptides produced by recombinant engineering.

More specifically, the hydrogenase-type enzymes, both those commonly known as "Fe-Ni" type and "Fe-only" type, that may be used in this invention may be isolated from hydrogen-activating bacteria belonging, for illustrative purposes and without this limiting the scope of the invention, to the following group:
- sulfur-reducing species, which include, albeit not exclusively, those from the *Desulfovibrio genre,*
- nitrogen-fixating species, which include, albeit not exclusively, those from the *Rhizobium, Bradyrhizobium, Azotobacter, Gloeocapsa* genres,
- methanogenic species, which include, albeit not exclusively, those from the *Methanobacterium, Methanococcus, Methanosarcina* or *Methanopyrus* genres,
- fermentative species, which include, albeit not exclusively, those from the *Bacillus, Clostridium* or *Escherichia* genres,
- chemolithotrophic species, which include, albeit not exclusively, those from the *Alcaligenes, Ralstonia* and *Aquifex hyperthermophillic* genres,
- photosynthetic species, which include, albeit not exclusively, those from the *Pyrococcus, Thermococcus* or *Thermotoga* genres.

Other hydrogenase-type enzymes that may be used in this invention may be isolated from hydrogen-metabolising algae, both prokaryotes and eukaryotes, belonging, for illustrative purposes and without this limiting the scope of the invention, to the following species: *Scenedesmus obliquus, Anabaena cilindrica* and *Anabaena variabilis, Anacystis nidulas, Synechocystis sp PCC 6803, Nostoc PCC73102* and *Synechococcus PCC6301.*

More broadly, the scope of this invention considers the use of hydrogenases produced from organisms belonging to the following taxonomic groups: *Crenarchaeota* and *Euryarchaeota* within the Archea domain; *Aquificae, Cyanobacteria, Firmicutes, Proteobacteria* and *Thermotoga* within the Bacteria domain; *Ciliophora, Parabasalidea, Chlorophyta* and *Fungi* within the Eukarya domain.

On the other hand, an enzyme may be used that has the hydrogenase activity modified in such a way that it has affinity motifs located on specific sites of the surface which allow for oriented immobilisation on the conducting electrode, conveniently modified with complementary affinity motifs to facilitate electrical communication between the enzyme and the electrode.

Therefore, a particular object of the invention is the biological electrode of the invention wherein the hydrogenase enzyme contains affinity motifs located on specific sites of the surface which allow for oriented immobilisation on the conducting electrode. For example, certain metal complexes have an affinity for histidine groups, which may be introduced in hydrogenase by means of genetic engineering techniques.

Moreover, these hydrogenases may be produced in native microbial species or from species modified by genetic engineering techniques or eventually produced in complex *in vitro* systems starting from gene sequences that are already known in the state of the art or that may be identified in the future by a technician skilled in the art.

The use of already-known enzymes with hydrogenase activity, or new hydrogenases, for the development of the biological electrode of this invention may be performed by a technician skilled in the art with the information described in this invention.

The term "conducting material", as used in this invention, refers to electricity-conducting materials that belong, for illustrative purposes and without this limiting the scope of the invention, to the following group: metallic material (gold, copper, silver or platinum) or carbonaceous material (glassy carbon, "basal" pyrolytic carbon, "edge" pyrolytic carbon, carbon thread and carbon fabric, amongst other types of conducting carbon), on the surface whereof primary amines may be introduced by means of different techniques that allow for anchoring with the enzymes.

Thus, another particular object of this invention is the biological electrode of the invention wherein the conducting electrode material used is a metallic material that belongs, for illustrative purposes and without this limiting the scope of the invention, to the following group: gold, copper, silver or platinum.

Another particular object of this invention is the biological electrode of the invention wherein the electrode material used is a carbonaceous material that belongs, for illustrative purposes and without this limiting the scope of the invention, to the following group: glassy carbon, "basal" pyrolytic carbon, "edge" pyrolytic carbon, carbon thread and carbon fabric.

A particular embodiment of this invention is the biological electrode of the invention wherein the hydrogenase-type enzyme is isolated from the *Desulfovibrio gigas* E.C.1.18.89.1 bacteria (the amino acid sequence of the enzyme used has the PDB reference code 1 H2A in the Protein Data Bank) and the electrode is gold (see example 1).

Another particular embodiment of this invention is the biological electrode of the invention wherein the hydrogenase-type enzyme is isolated from the *Desulfovibrio gigas* E.C.1.18.89.1 bacteria (the amino acid sequence of the enzyme used has the PDB reference code 1H2A in the Protein Data Bank) and the electrode is made of "edge" pyrolytic carbon (see example 2).

Another particular embodiment of the invention is the biological electrode of the invention composed of an electrode made of carbonaceous material modified with carboxyl groups and additionally modified with N,N-bis(carboxymethyl)-L-lysine, to form nitrilotriacetic-type complexes with Ni, Cu or Co ions (these metal complexes have an affinity for histidine groups), and a modified or mutated hydrogenase enzyme that comprises a histidine motif on suitable sites of the surface of the enzyme, thus allowing for correct immobilisation of the hydrogenase molecules with respect to the electrode. As an example of the methodology to prepare this biological electrode, a practical embodiment is cited which has been prepared with the ferredoxin-NADP+ reductase enzyme - as an enzyme model - on gold electrodes functionalised with Cu or Ni metal complexes with nitrilotriacetic chains and histidine pairs introduced, by means of genetic engineering techniques, in regions of the surface of the enzyme selected to produce, following immobilisation on the surface of the electrode, the desired orientations and, in an optimal orientation, with catalytic activity in the absence of external redox mediators (J. Madoz, J.M. Abad, J. Fernandez-Recio, M. Velez, L. Vazquez, C. Gomez-Moreno, V.M. Fernández. Modulation of electroenzymatic NADPH oxidation through oriented immobilization of ferredoxin:NADP reductase onto modified gold electrodes. Journal of the American Chemical Society (2000) 122: 9808-9817).

On the other hand, the biological electrode of the invention may be manufactured, produced or elaborated in different ways depending on whether the conducting electrode material is metal or carbon. Thus, another object of this invention is a method of producing the biological electrode of the invention with the conducting electrode made of carbon material, hereinafter method of producing a carbon electrode of the invention, based on the following steps:
a) modification of the carbon electrode by electroreduction of an aryl derivative of diazonium salts in an aprotic medium or an acid aqueous medium, which generates primary amino groups,
b) covalent binding of the hydrogenase molecules in phosphate buffer between 0.01 and 1.0 M, pH between 5.0 and 9.0, through its carboxyl groups activated with N-hydroxysuccinimide in the presence of soluble ethylcarbodiimide for a suitable period of time, and
c) subsequent washing of the electrode with phosphate buffer.
   Subsequently, the biological electrode thus produced is activated in a hydrogen atmosphere at ambient temperature prior to being used.

The functionalisation of carbon electrodes with a monolayer of amino groups, or carboxyl groups, may be performed by methods described by Saveant et al., by means of the electrochemical reduction of suitable aryl diazonium salts (Delamar, M., Hitmi, R., Pinson, J., Saveant, J.M., J. Am. Chem. Soc. 119, 201-207, 1992 Allongue, P.; Delamar, M.; Desbat, B.; Fagebaume, O.; Hitmi, R.; Pinson, J.; Saveant, J. M. J. Am. Chem. Soc. 1997, 119, 201-207).

Likewise, the functionalisation of the surface of carbon electrodes with carboxyl groups may be performed by other chemical methods. For example, by treating the carbon surfaces with oxygen plasma (J.I. Paredes, A. Martinez-Alonso and J.M.D. Tascón, J. Colloid Interf. Science 258, 276-282, 2003) or by direct oxidation with nitric acid or hydrogen peroxide (Detection of specific electronic interactions at the interface aromatic hydrocarbon-graphite by immersion calorimetry. B. Bachiller-Baeza, A. Guerrero-Ruiz and I. Rodriguez-Ramos. Studies on Surface Science and Catalysis, in press) or by combined chemical and electrochemical treatment consisting of electrochemical oxidation in the presence of potassium dichromate (J. Bianco, J. Haladjian and C. Bourdillon. J. Electroanalytical Chemistry, 293, 151-163 (1990).

Furthermore, starting from these electrodes functionalised with carboxyl groups, chemical modification with diamines (for example, ethylenediamine) may be performed to generate free terminal amino groups covalently bound to the carbon surface of the electrode.

Another particular object of this invention is the method of producing a carbon electrode of the invention wherein the aryl derivative of diazonium salts in point a) belongs, for illustrative purposes and without this limiting the scope of the invention, to the following group: nitrobenzyldiazonium salt and 2,5-dimethoxy-4-([4-nitrophenyl]azo)benzenediazonium.

In this invention, the pH value (within a range between 5.0 and 9.0) and the ionic strength (corresponding to a phosphate buffer molarity range between 0.01 and 1.0 M) during covalent immobilisation of the enzyme are critical in order to orient it appropriately and allow for the direct transfer of electrons to the electrode, having established some preferred conditions (see example 2). Thus, a particular embodiment of the invention is the method of producing the invention wherein the covalent binding step in b) is performed in 0.01 M phosphate buffer, pH 6.0, in the presence of 1 mM N-hydroxysuccinimide and 2 mM ethylcarbodiimide, for an incubation period of 90 minutes.

Another particular embodiment of the invention is a method of producing the carbon electrode wherein the electrode is made of "edge" pyrolytic carbon previously modified with the reagent tetrafluoroborate 4-nitrobenzenediazonium (according to the method described by P. Allonge et al. in the Journal of the American Chemical Society, 119, 201-207, 1997), which is put in contact with a 27 µM solution of purified hydrogenase from *Desulfovibrio gigas* in 0.01 M phosphate buffer, pH 6.0, in the presence of 1 mM N-hydroxysuccinimide and 2 mM ethylcarbodiimide, for an incubation period of 90 minutes in this solution, with subsequent washing of the electrode with phosphate buffer. This biological electrode is activated in a hydrogen atmosphere at ambient temperature for 5 hours.

Another particular object of this invention is a second method of producing the carbon electrode of the invention that is based on the modification of carbon material surfaces by plasma irradiation to generate carboxyls which, conveniently modified with diamines, for example in the presence of carbodiiimide, and eventually N-hydroxysuccinimide, lead to coatings with primary amines.

Another object of this invention is a method of producing the biological electrode of the invention with the metallic conducting electrode, hereinafter method of producing a metallic electrode of the invention, by the modification of the metal, gold, copper, silver, platinum for example, consisting of a coating with a monolayer of bifunctional alkylthiol molecules which, on one end, have thiol groups with chemisorption tendency and, on the other end, have functional groups that react with functional groups exposed on the hydrogenase enzyme for its oriented immobilisation (J. Madoz, B. Kuznetsov, J.L. Garcia, J. Medrano and V.M. Fernandez. Functionalization of gold surfaces for specific and oriented attachment of a fused b-Galactosidase and choline-receptor protein; Journal of the American Chemical Society (1997) 119: 1043- 1051; J. Madoz, J.M. Abad, J. Fernández-Recio, M. Velez, L. Vazquez, C. Gómez-Moreno & V.M. Fernández. Modulation of electroenzymatic NADPH oxidation through oriented immobilization of Ferredoxin:NADP* reductase onto modified gold electrodes. Journal of the American Chemical Society (2000) 122: 9808-9817; see example 1)).

The biological electrodes of the invention used as anodes make it possible to produce electrical energy from hydrogen in a typical fuel cell configuration; likewise, using these biological electrodes as cathodes it is possible to produce hydrogen from water in a typical electrochemical cell configuration.

Thus, another object of this invention is the use of the biological electrode of the invention, hereinafter use of the invention, in the manufacturing of a fuel cell anode or an electrochemical cell cathode. Similarly, a fuel cell or an electrolytic cell that comprise the biological electrode of the invention form a part of this invention.

### DESCRIPTION OF THE FIGURES

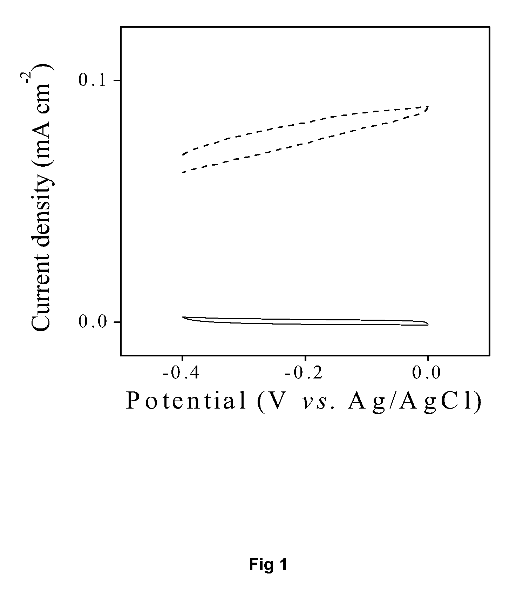

**Figure 1****. Cyclic voltammogram obtained in a gold metallic electrode.** This electrode has an electrochemical surface area of 0.18 cm2. The lower curve corresponds to a measurement performed in nitrogen (continuous curve) and the upper curve corresponds to the same electrode after 5 hours of incubation in a hydrogen atmosphere (dashed line).

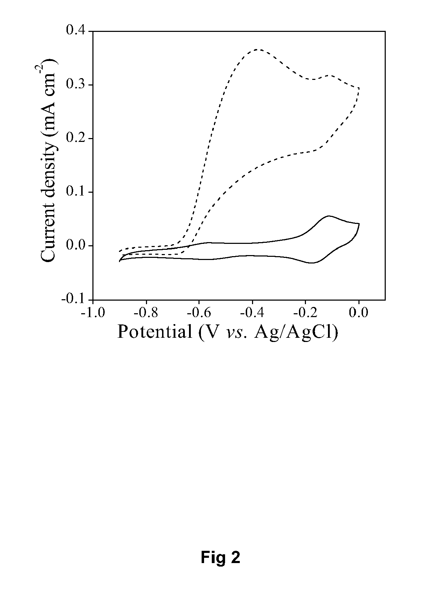

**Figure 2****.- Cyclic voltammogram of a pyrolytic carbon electrode bound to a hydrogenase enzyme.** The continuous line corresponds to a control electrode kept in nitrogen for 30 minutes hours prior to the electrochemical experiment, and the discontinuous line corresponds to a similar electrode after having activated the hydrogenase enzyme in hydrogen for five hours.

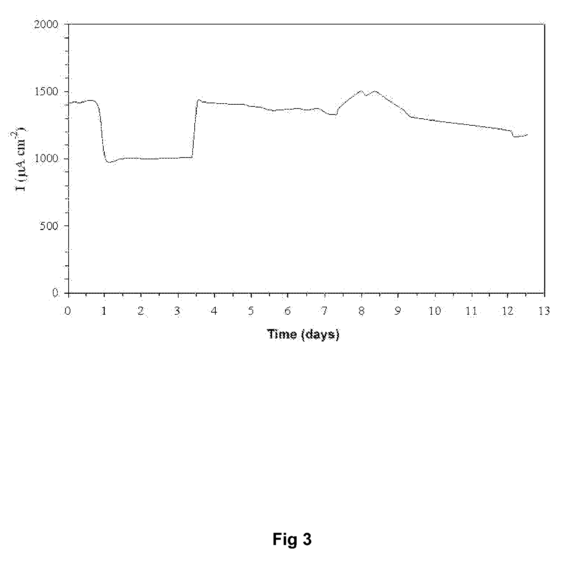

**Figure 3****.- Current density, measured as a function of time, of a hydrogenase-modified carbon nanotube electrode.** Chronoamperogram recorded at - 0.52 V compared to a calomel electrode, 2,500 rpm, 1 atm of H2 and 40°C, except between days 1 and 3.5, when the temperature was 20°C.

### EMBODIMENT EXAMPLES

### Example 1.- Biological electrode on gold metallic electrode.

A particular embodiment of this invention is the biological electrode of the invention wherein the hydrogenase-type enzyme is isolated from the *Desulfovibrio gigas* E.C.1.18.89.1 bacteria (aa sequence of the enzyme used or reference in the Protein Data Bank has the code PDB 1 H2A) and the electrode is polycrystalline gold in the form of thread, polished and washed as described in Fernández V.M. et al. (Journal of the American Chemical Society, 119, 1043-1051, 1997), which is sonicated in ethanol for 15 minutes. Subsequently, it is incubated in 1 mM cysteamine in 2:1 ethanol:water for 20 hours; following washing with 10 mM Hepes buffer, pH 7.6, it is submerged in a 0.8 micromolar solution of hydrogenase from *D. gigas* containing N-(3-dimethylaminopropyl)-N'-ethylcarbodiimide hydrochloride hydrate and 10 mM N-hydroxysuccinimide in Hepes buffer, pH 7.6, for 90 min at 25°C.

Subsequently, it is placed in a typical three-electrode electrochemical cell, the reference electrode being made of Ag/ClAg, the counter electrode being made of platinum and the working electrode being the hydrogenase-modified gold electrode. Cyclic voltammograms were performed with an Autolab 30 potentiostat under a controlled atmosphere at 25°C.

Figure 1 shows cyclic voltammograms obtained with this electrode, which has an electrochemical surface area of 0.18 cm2. The measurements were made in 100 mM phosphate buffer, pH 8.0, at a sweep rate of 20 mV/s and a temperature of 25°C. The lower curve corresponds to a measurement performed in nitrogen (continuous curve) and the upper curve corresponds to the same electrode after 5 hours of incubation in a hydrogen atmosphere (dashed line).

These gold biological electrodes have been functional both for the production of hydrogen (from protons and electrons supplied by the electrode) and the oxidation of hydrogen present in the solution, with the concomitant production of an electrical current, although the stability obtained in this specific embodiment is lower than that observed in the following example.

### Example 2.- Biological electrode in a pyrolytic carbon electrode.

In the preparation of functional hydrogenase electrodes, the use of electrodes made of carbonaceous materials, such as glassy carbon or pyrolytic carbon, has been researched. These electrodes, conveniently modified by means of chemical treatments, have allowed for lasting covalent binding of the hydrogenase molecules isolated from the *Desulfovibrio gigas* bacteria, purified according to the method described by E.C. Hatchikian et al. (Characterization of the periplasmic hydrogenase from Desulfovibrio gigas*.* E.C. Hatchikian, M. Brushi and J. leGall, Biochemical and Biophysical Research Communications 82, 451-461 (1978)).

In this example, a superficial layer of primary amines is created on pyrolytic carbon electrodes obtained from Pine Instruments, Inc. (USA), by electrochemical reduction of diazonium salts; in the example described, an "edge" pyrolytic carbon electrode (previously modified with the reagent tetrafluoroborate 4-nitrobenzenediazonium, according to the method described by P. Allonge et al. in the Journal of the American Chemical Society, 119, 201-207, 1997), is made to react with a 27 µM solution of purified hydrogenase from *Desulfovibrio gigas* in 0.01 M phosphate buffer, pH 6.0, in the presence of 1 mM N-hydroxysuccinimide and 2 mM ethylcarbodiimide. Following 90 minutes of incubation in this solution, the electrode is washed with phosphate buffer and, subsequently, it is placed in a typical three-electrode electrochemical cell, the reference electrode being made of Ag/ClAg, the counter electrode being made of platinum, and the working electrode being the hydrogenase-modified pyrolytic carbon electrode. Cyclic voltammograms were performed with an Autolab 30 potentiostat under a controlled atmosphere at 25°C.

Figure 2 shows cyclic voltammograms for the catalytic oxidation of hydrogen with the pyrolytic graphite electrodes covalently modified with hydrogenases following the procedure described above. The measurements were performed at 25°C, in 100 mM phosphate buffer, pH 8.0, with a sweep rate of 20 mV/s. The continuous line corresponds to a control electrode kept in a nitrogen atmosphere for 30 minutes hours prior to the electrochemical experiment. The dotted line corresponds to a cyclic voltammogram obtained with the hydrogenase electrode after having activated the hydrogenase enzyme in hydrogen for five hours; the cyclic voltammogram corresponding to the activated enzyme (dotted line) clearly shows this electrode's catalytic activity, which persists even after one week of work under operating conditions (Figure 2).

It is worth highlighting that no redox mediators have been added in any of these experiments. The results are conclusive as to the existence of important hydrogen oxidation currents catalysed by the hydrogenase enzyme in an optimal orientation for electronic communication with the surface of the electrode. Under the same conditions, those electrodes wherein the enzyme was not covalently bound did not give rise to any detectable current, not even in the presence of redox mediators. Those electrodes that were prepared in a similar manner to high ionic strength values did not lead to measurable currents.

A comparison was made between the currents measured, in the presence and in the absence of mediators, with electrodes prepared in a similar manner to those in the example but with different ionic strengths. The results shown below indicate that the activity increases as the ionic strength decreases.

| **Table 1. Relationship between the direct catalytic currents and that measured with methyl viologen as a mediator observed with hydrogenase-modified electrodes at different ionic strengths** | | | | |
|---|---|---|---|---|
| Ionic strength | 0.02 | 0.31 | 0.60 | 1.0 |
| _I_{cat} (dir)/ I_{cat} (meas)^{a} | 0.70 | 0.47 | 0.15 | 0.06 |

| | | | | |
|---|---|---|---|---|
| ^{a)} The catalytic current was measured at 0 V from cyclic voltammograms similar to those in Figure 2 after subtracting the currents measured in nitrogen, prior to activating the electrode with hydrogen for 5 hours. | | | | |

In this invention, the pH value (within a range between 5.0 and 9.0) and the ionic strength value (corresponding to a phosphate buffer molarity range between 0.01 and 1.0 M) during covalent immobilisation of the enzyme are critical in order to orient it appropriately and allow for the direct transfer of electrons to the electrode, which provides great stability to the electrocatalyst under the operating conditions.

### Example 3.- Biological electrode in a carbon nanotube electrode

Hydrogenase from *D. gigas* was covalently bound to a carbon nanotube electrode, with a geometric surface area of 0.017 cm2, in the same manner as in Example 2. In this case, the current densities measured are higher due to the electrode's greater roughness. The stability was measured continuously, at a fixed potential of -520 mV, against a calomel electrode, with an electrode rotation of 2,500 rpm, under a hydrogen current and at a temperature of 40°C, it being observed that, following two continuous weeks, 80% of the initial activity was maintained.

The rational basis for the method of immobilising the Fe-Ni hydrogenase, which is the object of this invention and whereof the enzyme from *Desulfovibrio gigas* may be considered a typical structure, is the presence of a strong dipolar moment in said enzyme's molecule as a consequence of an asymmetric distribution of charged amino acid residues on the surface of the enzyme. In particular, the protein's distal redox cluster (a [4Fe-4S] group wherethrough the enzyme transfers the electrons produced from oxidation of the hydrogen molecule) is surrounded by a crown of glutamic residues. This permanent dipole in the hydrogenase molecule turns out to be essential for the correct transfer of electrons to the cytochrome c3 molecule, the physiological acceptor of this type of hydrogenases, which exhibits a grouping of lysines the positive charges whereof complement the charges of the glutamic residues, thus facilitating a correct orientation of both proteins and an optimal transfer of electrons between them.

## Claims

1. Biological electrode **characterised in that** it contains a hydrogenase-type enzyme, Ni-Fe or "Fe-only" type, as a direct hydrogen oxidation catalyst, covalently bound, with a preferred orientation, to an electrode made of an electricity-conducting material through, respectively, carboxylic residues or amino residues exposed on the surface or introduced in the surface of said electrode.

2. Biological electrode, according to claim 1, **characterised in that** the hydrogenase-type enzyme is a hydrogenase isolated from hydrogen-activating microorganisms or produced by recombinant genetic engineering.

3. Biological electrode, according to claim 2, **characterised in that** the hydrogenase-type enzyme is isolated from hydrogen-activating bacteria belonging to the following group:
- sulfur-reducing species, which include, albeit not exclusively, those from the *Desulfovibrio* genre,
- nitrogen-fixating species, which include, albeit not exclusively, those from the *Rhizobium, Gloeocapsa* genres,
- methanogenic species, which include, albeit not exclusively, those from the *Methanobacterium, Methanococcus, Methanosarcina* or *Methanopyrus* genres,
- fermentative species, which include, albeit not exclusively, those from the *Bacillus, Clostridium* or *Escherichia* genres,
- chemolithotrophic species, which include, albeit not exclusively, those from the *Alcaligenes, Ralstonia* and *Aquifex hyperthermophillic* genres,
- photosynthetic species, which include, albeit not exclusively, those from the *Chromatium* or *Rhodobacter* or *Thiocapsa* genres), hyperthermophillic species (which include, albeit not exclusively, those from the *Pyrococcus* or *Thermococcus* or *Thermotoga* genres).

4. Biological electrode, according to claim 2, **characterised in that** the hydrogenase-type enzyme is isolated from hydrogen-metabolising algae, both prokaryotes and eukaryotes, belonging to the following species: *Scenedesmus obliquus, Anabaena cilindrica* and *Anabaena variabilis, Anacystis nidulas, Synechocystis sp PCC 6803, Nostoc PCC73102* and *Synechococcus PCC6301.*

5. Biological electrode, according to claim 1, **characterised in that** the hydrogenase-type enzyme is an enzyme with hydrogenase activity modified with affinity motifs located on specific sites of the surface thereof, which allow for oriented immobilisation on the conducting electrode, conveniently modified with complementary affinity motifs.

6. Biological electrode, according to claim 5, **characterised in that** the modification consists of a histidine group.

7. Biological electrode, according to claim 1, **characterised in that** the conducting material electrode belongs to the following group: metallic material or carbonaceous material.

8. Biological electrode, according to claim 7, **characterised in that** the metallic material belongs to the following group: gold, copper, silver or platinum.

9. Biological electrode, according to claim 7, **characterised in that** the carbonaceous material belongs to the following group: glassy carbon, "basal" pyrolytic carbon, "edge" pyrolytic carbon, carbon thread, carbon fabric, microporous carbon, carbonous molecular sieves, carbon nanotubes, carbon nanofibres.

10. Biological electrode, according to claim 1, **characterised in that** the hydrogenase-type enzyme is the enzyme isolated from the *Desulfovibrio gigas* E.C.1.18.89.1 bacteria (the amino acid sequence of the enzyme used has the PDB reference code 1H2A in the Protein Data Bank) and the conducting electrode is made of gold.

11. Biological electrode, according to claim 1, **characterised in that** the hydrogenase-type enzyme is the enzyme isolated from the *Desulfovibrio gigas* E.C.1.18.89.1 bacteria (the amino acid sequence of the enzyme used has the PDB reference code 1H2A in the Protein Data Bank) and the conducting electrode is made of "edge" pyrolytic carbon.

12. Biological electrode, according to claim 1, **characterised in that** it is composed of an electrode made of carbonaceous material modified with carboxyl groups, and additionally modified with N,N-bis(carboxymethyl)-L-lysine, to form nitrilotriacetic-type complexes with Ni, Cu or Co ions (these metal complexes have an affinity for histidine groups), and a modified or mutated hydrogenase enzyme that comprises a histidine motif on suitable sites of the surface of the enzyme.

13. Method of producing the biological electrode, according to claim 1, **characterised in that** the conducting electrode is made of carbonaceous material and it comprises the following steps:
a) modification of the carbon electrode by electroreduction of an aryl derivative of diazonium salts in an aprotic medium or an acid aqueous medium, which generates primary amino groups,
b) covalent binding of the hydrogenase molecules in phosphate buffer between 0.01 and 1.0 M, pH between 5.0 an 9.0, through its carboxyl groups activated with N-hydroxysuccinimide in the presence of soluble ethylcarbodiimide for an adequate period of time, and
c) subsequent washing of the electrode with phosphate buffer.

14. Method of producing the biological electrode, according to claim 13, **characterised in that** the aryl derivative of diazonium salts in point a) belongs to the following group: nitrobenzyldiazonium salt and 2,5-dimethoxy-4-([4-nitrophenyl]azo)benzenediazonium.

15. Method of producing the biological electrode, according to claim 13, **characterised in that** the covalent binding step is performed in 0.01 M phosphate buffer, pH 6.0, in the presence of 1 mM N-hydroxysuccinimide and 2 mM ethylcarbodiimide, for an incubation period of 90 minutes.

16. Method of producing the biological electrode, according to claim 13, **characterised in that** the conducting electrode is an "edge" pyrolytic carbon electrode previously modified with the reagent tetrafluoroborate 4-nitrobenzenediazonium, which is put in contact with a 27 µM solution of purified hydrogenase from *Desulfovibrio gigas* in 0.01 M phosphate buffer, pH 6.0, in the presence of 1 mM N-hydroxysuccinimide and 2 mM ethylcarbodiimide, for 90 minutes of incubation in this solution, with subsequent washing of the electrode with phosphate buffer.

17. Method of producing the biological electrode, according to claim 13, **characterised in that** the modification of the surface of the carbon electrode in a), specifically made of carbonaceous material, is performed by means of plasma irradiation in order to generate carboxyls which, conveniently modified with diamines, in the presence of carbodiiimide, and eventually N-hydroxysuccinimide, coat the electrode with primary amines.

18. Method of producing the biological electrode, according to claim 1,
**characterised in that** the conducting electrode is metallic and modification of the metallic electrode is performed by coating with a monolayer of bifunctional alkylthiol molecules which have, on one end, thiol groups with chemisorption tendency and, on the other end, functional groups that react with functional groups exposed on the hydrogenase enzyme for the oriented immobilisation thereof.

19. Method of producing the biological electrode, according to claim 18, **characterised in that** the metallic electrode is made of gold.

20. Use of the biological electrode, according to claims 1 to 12, in the manufacturing of a fuel cell anode or an electrochemical cell cathode.

21. Fuel cell **characterised in that** it comprises a biological electrode according to claims 1 to 12.

22. Electrolytic cell **characterised in that** it comprises a biological electrode according to claims 1 to 12.
